(19) Europäisches Patentamt
European Patent Office
Office européen des brevets

(11) **EP 4 385 530 A1**

(12) **EUROPEAN PATENT APPLICATION**

(43) Date of publication:
**19.06.2024 Bulletin 2024/25**

(21) Application number: **22214186.3**

(22) Date of filing: **16.12.2022**

(51) International Patent Classification (IPC):
*A61L 27/16* (2006.01)     *A61L 27/22* (2006.01)
*A61L 27/24* (2006.01)     *A61L 27/38* (2006.01)
*A61L 27/50* (2006.01)     *A61L 27/52* (2006.01)
*B33Y 70/00* (2020.01)     *B33Y 80/00* (2015.01)

(52) Cooperative Patent Classification (CPC):
(C-Sets available)
**A61L 27/38; A61L 27/16; A61L 27/222;
A61L 27/24; A61L 27/50; A61L 27/52; B33Y 70/00;
B33Y 80/00** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universiteit Gent
9000 Gent (BE)**

(72) Inventor: **The designation of the inventor has not
yet been filed**

(74) Representative: **Arnold & Siedsma
Bezuidenhoutseweg 57
2594 AC The Hague (NL)**

(54) **SCAFFOLDS FOR TISSUE ENGINEERING, MOULDS FOR CASTING SAID SCAFFOLDS AND
METHODS THEREOF**

(57) The present invention relates to a mould for casting a crosslinked 3-dimensional scaffold for tissue engineering, a method of manufacturing a crosslinked scaffold and a crosslinked 3-dimensional scaffold.

EP 4 385 530 A1

(52) Cooperative Patent Classification (CPC): (Cont.)

C-Sets
**A61L 27/16, C08L 29/04**

**Description**

FIELD OF THE INVENTION

The present invention relates to a mould for casting a crosslinked 3-dimensional scaffold for tissue engineering, a method of manufacturing a crosslinked scaffold and a crosslinked 3-dimensional scaffold.

BACKGROUND TO THE INVENTION

Since its emergence in the mid-1980s, tissue engineering has continued to evolve as an exciting and multidisciplinary field aiming to develop biological substitutes to restore, replace or regenerate defective tissues. Scaffolds, typically made of polymeric biomaterials, provide the structural support for cell attachment and subsequent tissue development.

Over the last decade, development of fabrication technologies for porous macroscaffolds has been an intensive area of research. In general, these technologies can be classified into (1) processes using porogens in biomaterials, (2) solid free-form or rapid prototyping technologies and (3) techniques using woven or non-woven fibers. Techniques belonging to (1) and (2) are the most used.

In (1), solid materials either in solids or dissolved in solvents, are incorporated with porogens, which could be gases such as carbon dioxide, liquids such as water or solids such as paraffin. The mixtures are processed and then cast or extruded, and then the porogens are removed leaving behind a porous structure in the scaffold. Example techniques include solvent casting and particulate leaching, gas foaming, freeze-drying and phase separation. Solvent casting has however the disadvantage that geometry and pore sizes cannot be predefined.

In (2), hierarchical porous structures are manufactured by sequential delivery of material and/or energy needed to bond the materials to preset points in space. Some solid free-form fabrication technologies such as selective laser sintering, stereolithography and 3D printing depend on precise delivery of light or heat energy in a scanner system to points of space in the material bed so as to bond or crosslink the materials to give solid structures in an otherwise soluble bed of materials. Some solid free-form fabrication technologies such as wax printing rely on simultaneous delivery of solid materials and removable supporting materials in a layer-by-layer printing manner. Two notable examples of technologies belonging to this category are Direct and Indirect 3D printing.

In Direct 3D printing, the scaffold and the cells encapsulated in a bio-ink are 3D printed. This has been applied as a standard method to obtain 3D scaffolds combining biomaterials and cell encapsulation. However, a disadvantage of this technique is that the bio-inks need to have certain characteristics to enable 3D printing. Due to this, the direct printing technique allows for the use of a limited number of materials. A further disadvantage of the present technique is that shear stress is applied to the encapsulated cells during printing.

Indirect 3D printing instead requires the use of a mould, the scaffold is obtained therefrom, after which the mould is removed. A disadvantage of the present technique is that the removal of the mould cannot be performed in the presence of cells due to the use of cytotoxic solvents to remove the sacrificial mould. Moreover, the casting of the scaffold results in the formation of air bubbles entrapped inside the mould, thereby requiring the mould and the scaffold to be subjected to vacuum treatment to obtain a uniform distribution of the material. A disadvantage of this method is thus that it can only be applied for cell seeding experiments, instead of cell encapsulation, while the latter is crucial to ensure a homogeneous cell distribution.

Based on the disadvantages highlighted above, it is clear that there is an industry need for novel methods for the formation of scaffolds for tissue engineering. The present invention aims at providing an indirect 3D printing method of scaffolds for tissue engineering, overcoming the drawbacks of the prior art.

SUMMARY OF THE INVENTION

According to a first aspect, the present invention pertains to a mould for casting a crosslinked 3-dimensional scaffold for tissue engineering, wherein the mould is for receiving a crosslinkable scaffold precursor in a liquid state, and wherein the mould is made of a water-soluble material. The present invention provides for several advantages, in particular, the mould can be removed with a solution comprising water. Further, an advantage of the present embodiment is that the mould can be filled with low viscous materials, providing minimal pressure/shear stress to cells encapsulated in the crosslinkable precursor solution during injection.

According to an embodiment of the present invention, the water-soluble material has a solubility in water at 37°C from 0.001 g/ml to 1 g/ml, preferably from 0.05 g/ml to 0.75 g/ml, more preferably 0.25 g/ml to 0.5 g/ml. An advantage of the present embodiment is that a solubility according to the present embodiment allows for the dissolution of the mould whilst minimal to no cytotoxicity to encapsulated cells is provided. Further, an advantage is that the present embodiment allows the use of biocompatible water-based solvents to dissolve the mould.

According to yet another embodiment of the present invention, the mould is of a material having a total transmittance (Total transmittance = specular Transmittance + diffuse Transmittance) of at least 80% at a wavelength from 300nm to 800nm upon contact with the crosslinkable scaffold precursor, preferably a total transmittance of at least 85%, preferably at least 90%, more preferably at least 95%. An advantage of the present embodiment is that the crosslinkable scaffold precursor to be provided to the mould can be irradiated with UV-vis light through the mould itself, thereby allowing the use of a scaffold precursor that can be crosslinked by means of UV-vis radiation. By utilizing a mould according to the present embodiment the UV-vis radiation can be provided from various directions, thereby allowing a more homogeneous crosslinking.

According to an embodiment of the present invention, the water-soluble material comprises PVA (Polyvinyl alcohol). An advantage of the present embodiment is that upon contact with the crosslinkable scaffold precursor the PVA becomes even more translucent, thereby facilitating the passage of light and hence facilitating irradiation of the crosslinkable scaffold precursor.

According to an embodiment of the present invention, the mould has a minimum thickness $T_{min}$, measured as the shortest distance from a point A on an inner surface to a point B on an outer surface, wherein $T_{min}$ is from 100 to 1500$\mu$m, preferably from 150 to 1250$\mu$m, more preferably from 250 to 1000$\mu$m. An advantage of the present embodiment is that the dissolution of the mould and the irradiation of the crosslinkable scaffold precursor is more efficient.

According to an embodiment of the present invention, the mould comprises a pouring cup adapted to receive a crosslinkable scaffold precursor. It has been found that a pouring cup is advantageous in that it facilitates the deposition of the crosslinkable scaffold precursor in the mould, e.g. by pipetting the crosslinkable scaffold precursor through said pouring cup. Further, the pouring cup allows the filling of the mould from the bottom up, thereby minimizing the formation of air bubbles during injection and evenly spreading the material.

In a further aspect, the present invention pertains to a method of manufacturing of a crosslinked scaffold for tissue engineering comprising the steps of:

a) providing a mould of a water-soluble material as described in any one embodiment of the present invention;
b) providing a crosslinkable scaffold precursor in a liquid state;
c) casting the crosslinkable scaffold precursor into the mould provided at step a), thereby obtaining a casted crosslinkable scaffold precursor;
d) crosslinking the casted crosslinkable scaffold precursor obtained at step c), thereby obtaining the crosslinked scaffold precursor,
e) providing a solvent adapted to dissolve the mould provided at step a);
f) removing the mould from the crosslinked scaffold at step d) by means of dissolution of the mould provided at step a) with the solvent provided at step e), thereby obtaining the crosslinked scaffold.

An advantage of the present embodiment is that it provides for crosslinked scaffolds in a really efficient way, as several scaffolds can be obtained in a few minutes, several materials can be assessed in parallel. Further, the present method allows for the formation of scaffolds having 3D structures highly effective for cell encapsulation. Furthermore, as the material combined with cells can be pipetted into the sacrificial mould, lower pressure/shear stress will be placed on the encapsulated cells. The materials can still be crosslinked as they would in a 2D setting. Moreover, as multiple scaffolds can be made in parallel in a few minutes, the dark cytotoxicity of unreacted compounds can be significantly diminished. Conventional additive manufacturing, such as extrusion-based 3D printing is also dependent on several parameters, such as f.e. environment (temperature,..). The method of the present invention decreases the influence of these parameters that could affect the reproducibility of the scaffolds obtained at a certain moment. The present method allows the manufacturing of more complex designs compared to methods in the state of the art. Further, the present method overcomes limitations associated with the use of non-biocompatible solvents, e.g. chloroform, to dissolve the mould, and also does not require the use of for vacuum treatment.

According to an embodiment of the present invention, at step b) the crosslinkable scaffold precursor comprises one or more of: cells, stem cells, organoids, spheroids, cell clusters, co-cultures. An advantage of the present embodiment is that compared to extrusion based bioprinting, cells will not experience a high level of shear stress, furthermore, any clustered cells will not negatively impact the printing parameters f.e. printing pressure, and thus the reproducibility of the constructs.

According to an embodiment of the present invention, for the method, at step b), the crosslinkable scaffold precursor has a water content from 2.5 to 15 w/v%, preferably from 5 to 12.5, more preferably from 5 to 10. It was found that the present embodiment is particularly useful in facilitating the irradiation of the crosslinkable scaffold precursor, as already upon contact with the crosslinkable scaffold precursor the transmittance of the mould material is increased, thereby allowing more light to pass through the mould, improving crosslinking.

According to an embodiment of the present invention, at step a) the mould is made of a material transparent or translucent after the crosslinkable scaffold precursor has been received by the mould, thereby allowing radiation, preferably UV-vis radiation, to reach the crosslinkable scaffold precursor. An advantage of the present embodiment is that by increasing light transmittance, lower irradiation times will potentially be needed to fully crosslink the scaffold.

According to an embodiment of the present invention, at step b) the crosslinkable scaffold precursor has a viscosity from 0.05 to 10 mPa.s, preferably from 0.5. to 1.5 mPa.s. An advantage of the present embodiment is that it facilitates the injection of the crosslinkable scaffold precursor into the mould.

According to yet a further embodiment of the present invention, at step d) the crosslinking is performed from about 1 to 30 min, preferably from about 1 to 20 min. The present embodiment was found advantageous because it provides for a higher biocompatibility as both long light exposure times and the presence of unreacted monomers can negatively impact cell behavior and survival.

In a further aspect, the present invention pertains to a crosslinked 3-dimensional scaffold for tissue engineering obtainable from a mould according to any embodiment of the present invention, or by means of the method according to the present invention, comprising encapsulated cells. An advantage of the present aspect that the scaffold provides for good cell availability.

In yet a further aspect, the present invention pertains to the use of the mould according to the present invention or of the scaffold according to the present invention in the context for tissue engineering purposes. According to a preferred embodiment of the present invention, the use is for cell encapsulation purposes.

BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

**Figures 1A, 1B and 1C,** also abbreviated as **Fig. 1A, 1B,** and **1C** illustrate an example of a CAD drawing of a mould in accordance with the present invention **(Fig. 1A),** the 3D printed mould **(Fig. 1B),** and an obtained scaffold for tissue engineering therefrom **Fig. 1C,** bottom, beside a mould **Fig. 1C,** top.

**Figure 2A and 2B,** also abbreviated as **Fig. 2A and 2B,** illustrate the mass swelling ratio **(Fig. 2A)** and the Young's modulus **(Fig. 2B)** of a variety of tested scaffolds obtained according to the present invention.

**Figure 3,** also abbreviated as **Fig. 3,** illustrates a photo of cell encapsulated scaffolds in accordance with the present invention.

**Figure 4,** also abbreviated as **Fig. 4,** illustrates measurements of cell viability for various hydrogels (GelMA, GelNBSH, RCPMA, RCPNBSH) comprising encapsulated cells.

**Figure 5,** also abbreviated as **Fig. 5,** illustrates bar charts of cell viability based on the assay also described in **Fig. 4.**

**Figure 6,** also abbreviated as **Fig. 6,** illustrates MTS of hydrosoluble materials.

DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. When describing the compounds of the invention, the terms used are to be construed in accordance with the following definitions, unless a context dictates otherwise.

The term "about" or "approximately" as used herein when referring to a measurable value such as a parameter, an amount, a temporal duration, and the like, is meant to encompass variations of +/- 10 % or less, preferably +/- 5 % or less, more preferably +/- 1 % or less, and still more preferably +/- 0.1 % or less of and from the specified value, insofar such variations are appropriate to perform in the disclosed invention. It is to be understood that the value to which the modifier "about" or "approximately" refers is itself also specifically, and preferably, disclosed.

As used in the specification and the appended claims, the singular forms "a", "an", and "the" include plural referents unless the context clearly dictates otherwise. By way of example, "a nanofiber" means one nanofiber or more than one nanofiber.

According to a first aspect, the present invention pertains to a mould for casting a crosslinked 3-dimensional scaffold for tissue engineering, wherein the mould is for receiving a crosslinkable scaffold precursor in a liquid state, and wherein the mould is made of a water-soluble material. The present invention provides for several advantages, in particular, the mould can be removed with a solution comprising water i.e water-based. Further, an advantage of the present embodiment is that the mould can be easily removed by means of various water containing liquids, also referred to as solvents, providing minimal cytotoxicity to cells encapsulated in the crosslinkable scaffold precursor. In particular, the solvent for the dissolution of the mould can be and is not limited to, any biocompatible sterile liquid used in a cell setting.

An advantage of the present invention is that the mould can be dissolved with water after it was 3D printed, thereby allowing the formation of a scaffold. The present invention has the advantage of allowing the use of a solvent bio compatible with cells, so that the crosslinkable scaffold precursor can be provided to comprise e.g. encapsulated cells.

By means of the term "crosslinkable scaffold precursor", reference is made to a compound or mixture capable of being crosslinked from which the scaffold according to the present invention can be obtained. In the context of the present invention, a variety of crosslinkable scaffold precursors can be used. For some embodiments of the present invention, the crosslinkable scaffold precursor is biocompatible. Should be clear to the skilled in the art that the crosslinkable scaffold precursors is preferably of a viscosity allowing to be poured/pipetted into a mould.

A variety of crosslinkable scaffold precursor are suitable to put the present invention into practice, such as, and not limited to: methacrylated gelatin solution, modified gelatin (f.e methacrylamide modified gelatin, norbornene-modified gelatin, thiolated gelatin, bifunctional gelatin,...), modified collagen (f.e. methacrylamide modified collagen), modified hyaluronic acid, modified dextran (norbornene-modified dextran, methacrylamide modified dextran), (recombinant) peptides and any combination thereof.

By means of the term "crosslinked scaffold precursor", reference is made to the crosslinked scaffold precursor in a crosslinked state, i.e. after it has been crosslinked.

By means of the term "crosslinked scaffold", reference is made to a scaffold according to the present invention which has been crosslinked at least partially.

According to yet an embodiment of the present invention, the mould is of a material having a total transmittance of at least 80% at a wavelength from 300nm to 800nm upon contact with the crosslinkable scaffold precursor, preferably a total transmittance of at least 85%, preferably at least 90%, more preferably at least 95%. An advantage of the present embodiment is that the crosslinkable scaffold precursor to be provided to the mould can be irradiated with UV-vis light through the mould itself, thereby allowing the use of a scaffold precursor that can be crosslinked by means of UV-vis radiation. By utilizing a mould according to the present embodiment the UV-vis radiation can be provided from various directions, thereby allowing a more homogeneous crosslinking. The total transmittance can be calculated according to various methods in the state of the art, for example, in the measurements carried out the total transmittance was measured by means of an UV-Vis spectrophotometer on the films of the materials measured, for a wavelength of the light beam ranged from 300 nm to 800 nm. The loss in transmittance was calculated compared to a blank measurement.

The mould according to the present invention can be made of a variety of different water soluble materials, having different water solubilities. According to an embodiment of the present invention, the water-soluble material has a solubility in water at 37ºC from 0.001 g/ml to 1 g/ml, preferably from 0.05 g/ml to 0.75 g/ml, more preferably 0.25 g/ml to 0.5 g/ml. An advantage of the present embodiment is that a solubility according to the present embodiment allows for the dissolution of the mould whilst minimal cytotoxicity to encapsulated cells is provided. Further, an advantage is that the present embodiment allows the use of biocompatible water based solvents to dissolve the mould.

Mould materials suitable to carry out the present invention are those water-soluble, such as, and not limited to materials comprising PVA and Butenediolvinylalcohol-co-polymer (Hydrosoluble). According to an embodiment of the present invention, the water-soluble material comprises PVA.

An advantage of the present embodiment is that upon contact with the crosslinkable scaffold precursor the PVA comprising water-soluble material becomes even more translucent, thereby facilitating the passage of light and hence facilitating irradiation of the crosslinkable scaffold precursor.

According to an embodiment of present invention, the water-soluble material comprises PVA at a concentration of at least 90%, preferably at least 91%, more preferably at least 92%, preferably at least 93%, preferably at least 94%, preferably at least 95%, preferably at least 96%, preferably at least 97%, preferably at least 98%, preferably at least 99%, preferably at least 100%. According to a specific embodiment of the present invention, the water-soluble material comprises PVA at a concentration of at least 96%.

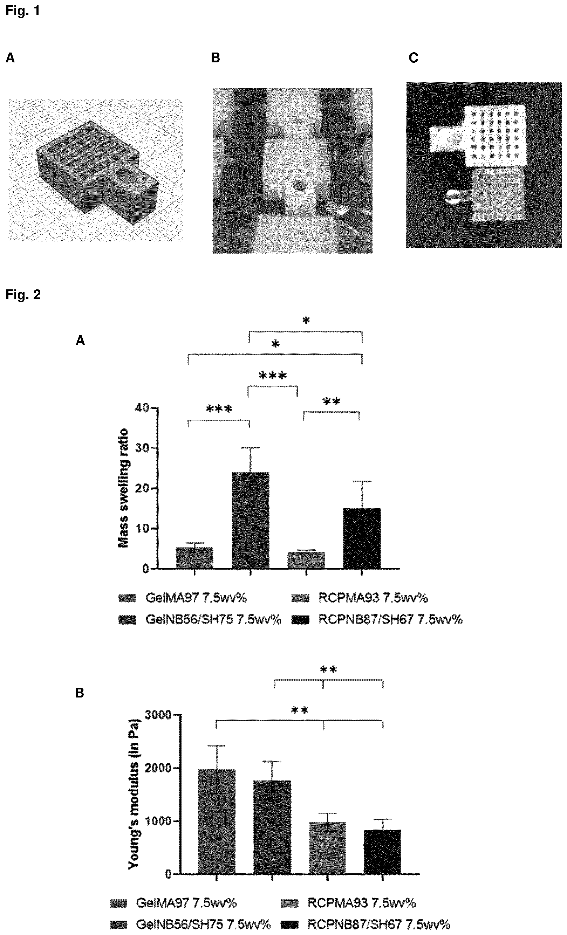

Fig. 1A illustrates an example of a CAD drawing of a mould in accordance with the present invention, wherein the mould is of a cubical shape and comprises a top face which at least partially open, and a spout, also referred to as pouring cup, from which the crosslinkable scaffold precursor could be pipetted. Fig. 1B illustrates a 3D printed mould made of PVA on basis of the CAD drawing of Fig. 1A. Fig. 1C further illustrates a scaffold obtained after crosslinking the crosslinkable scaffold precursor, see bottom of Fig. 1C. The scaffold thereby obtain can then be utilized for tissue engineering purposes. The scaffolds in Fig. 1C comprises encapsulated cells.

Even though the mould and then the scaffold as described in Fig. 1 are approximately cubical in shape, the mould and the scaffold according to the present invention can be provided in various shapes, such as cylindrical, parallelepipedal, cubical etc..

According to an embodiment of the present invention, the mould comprises:

- at least one opening
- an inner surface having a negative imprint of the crosslinked scaffold;
- an outer surface.

It was found beneficial, according to an embodiment of the present invention, to have a mould with a face which is at least partially open, such as the top face. This is to maximize light exposure on one hand, as well as giving a quicker way of the water reaching the inner struts, instead of first having to dissolve all the walls. A further advantage is that air bubbles can be more easily removed. The mould of the present invention is provided to retain the liquid crosslinkable scaffold precursor, so that it can be crosslinked according to various techniques in the state of the art. Preferably the mould of the present invention has a minimum thickness $T_{min}$, measured as the shortest distance from a point A on the inner surface of the mould to a point B on the outer surface of the mould , wherein $T_{min}$ is from 100 to 1500μm, preferably from 150 to 1250μm, more preferably from 250 to 1000um. An advantage of the present embodiment is that the dissolution of the mould and the irradiation of the crosslinkable scaffold precursor is more efficient.

The mould according to the present invention can be provided with an inner surface having a variety of different patterns, such as a mesh pattern. The inner surface can also provide for porous structures in the final manufactured crosslinked scaffold having different pore size, which are selected depending on the desired application of said scaffold. It was nevertheless, found advantageous to provide use scaffolds having pore sizes anywhere between 150-1000μm.

According to an embodiment of the present invention, the mould comprises a pouring cup adapted to receive a crosslinkable scaffold precursor and provide the crosslinkable scaffold precursor to the inner surface. By means of the term "pouring cup", reference is made to a tube or lip projecting from the scaffold. The pouring cup according to the present invention is designed so that the crosslinkable liquid is deposited to the bottom of the mould first, providing minimal contact between the inner struts in the mould. It has been found that a pouring cup is advantageous in that it facilitates the deposition of the crosslinkable scaffold precursor in the mould, e.g. by pipetting the crosslinkable scaffold precursor through said pouring cup.

In a further aspect, the present invention pertains to a method of manufacturing of a crosslinked scaffold for tissue engineering comprising the steps of:

a) providing a mould of a water-soluble material as described in any one embodiment of the present invention; and
b) providing a crosslinkable scaffold precursor in a liquid state;

The crosslinkable scaffold precursor can be provided with a variety of biological entities present therein, such as, and not limited to, one or more of: cells, stem cells, organoids, spheroids, cell clusters, co-cultures.

Further, the method according to the present invention comprises:
c) casting the crosslinkable scaffold precursor into the mould provided at step a), thereby obtaining a casted crosslinkable scaffold precursor;

By means of casting, references is made to the act of pouring a liquid, in this case the crosslinkable scaffold precursor, into the mould. Step c) can be performed by pipetting the crosslinkable scaffold precursor into the mould, for example from an opening, even partial, provided at the top or any other location, or a pouring cup, that facilitates the operation. Further, the pouring cup allows the filling the mould from the bottom, thereby minimizing the formation of air bubbles during injection and evenly spreading the material.

Further, the method comprises:
d) crosslinking the casted crosslinkable scaffold precursor obtained at step c), thereby obtaining the crosslinked scaffold precursor,
Step d) according to the present method can be accomplished by means of various crosslinking techniques in the state of the art and based on the type of crosslinkable scaffold precursor. Photocrosslinking is a crosslinking technique advantageous for the material tried, nevertheless, other materials can be crosslinked without the need of irradiation f.e. enzymatic, redox initiated, etc. For example, the crosslinkable scaffold precursor, f.e. methacrylamide modified gelatin in a 5 w/v% (GelMA 5w/v%) solution, can be prepared and mixed with the desired cell line and cell density. Following injection, the GelMA 5wv% can then be crosslinked in the presence of a photoinitiator.
Further, the method comprises
e) providing a solvent adapted to dissolve the mould provided at step a);

Hence, step e) comprises contacting the mould of a water soluble material with a solvent comprising adapted to dissolve said mould. The solvent hereby defined serves the purpose of removing the mould from the crosslinked scaffold precursor, obtained at step d). Various solvents can be used in accordance with the present invention, such as, and not limited to; balanced salt solutions (PBS), basal media (MEM, DMEM,...), HEPES buffer, cell culture media (DMEM+FBS+P/S), FBS. In case cells or other living material is encapsulated within the crosslinkable scaffold precursor, it is important that the solvent is not cytotoxic, so that minimal stress is provided.
Finally, the method of the present invention comprises:
f) removing the mould from the crosslinked scaffold at step d) by means of dissolution of the mould provided at step a) with the solvent provided at step e), thereby obtaining the crosslinked scaffold.
An advantage of the present embodiment is that it provides for crosslinked scaffolds in a really efficient way, as several scaffolds can be obtained in a few minutes. In other words, the present method is high-throughput, as it has allowed for example the fabrication of more than 30 scaffolds per hour, allowing for the development of multiple scaffolds from different crosslinkable scaffold precursors, or obtained via different crosslinking conditions, in parallel. Furthermore, since the mould contains the solution and holds it in place before crosslinking occurs, a better CAD/CAM mimicry can be obtained as flow of the bio-ink precursor as well as overhang is minimized. As PLA can be used to create a support structure for the PVA mould (which can be washed away with f.e. chloroform prior to hydrogel precursor injection, overhanging constructs can more easily be obtained than in case of direct extrusion based bioprinting f.e., while maintaining the potential towards cell encapsulation compared to PLA sacrificial moulds.

Further, the present method allows for the formation of scaffolds having 3D structures highly effective for cell encapsulation. Low viscous crosslinkable scaffold precursors can also be printed through this technique, and further higher resolution can be obtained with less shear stress on the cells, which is very important for cell survival.

Furthermore, as the material combined with cells can be pipetted into the sacrificial mould, lower pressure/shear stress will be placed on the encapsulated cells. The materials can still be crosslinked as they would in a 2D setting. Moreover, as multiple scaffolds can be made in parallel in a few minutes, the dark cytotoxicity of unreacted compounds can be significantly diminished. Conventional additive manufacturing, such as extrusion-based 3D printing is also dependent on several parameters, such as f.e. environment (temperature,..). The method of the present invention decreases the influence of these parameters that could affect the reproducibility of the scaffolds obtained at a certain moment. Hence, more reproducible scaffolds can be fabricated. The present method allows the manufacturing of more complex designs compared to methods in the state of the art. Further, the present method overcomes limitations associated with the use of non-biocompatible solvents, e.g. chloroform, to dissolve the mould, and also does not require the use of vacuum treatment.

According to an embodiment of the present invention, at step b) the crosslinkable scaffold precursor comprises one or more of: cells, stem cells, organoids, spheroids, cell clusters, co-cultures.

According to an embodiment of the present invention, for the method, at step b), the crosslinkable scaffold precursor has a water content from 2.5 to 15 w/v%, preferably from 5 to 12.5, more preferably from 5 to 10.

According to an embodiment of the present invention, at step a) the mould is made of a material transparent or translucent after the crosslinkable scaffold precursor has been received by the mould, thereby allowing radiation, preferably UV-vis radiation, to reach the crosslinkable scaffold precursor

According to an embodiment of the present invention, at step b) the crosslinkable scaffold precursor has a viscosity from 0.05 to 10 mPa.s, preferably from 0.5. to 1.5 mPa.s.

According to yet a further embodiment of the present invention, at step d) the crosslinking is performed from about 1 to 30 min, preferably from about 1 to 20 min. The present embodiment is particularly advantageous in case the crosslinkable scaffold precursor is crosslinked by means of UV, as it provides for the best cell viability results when irradiation time with UV-A can be minimized. It was found that when visible light needs to be provided to crosslink the crosslinkable scaffold precursor, the crosslinking time is affecting less the viability and the formation of the scaffold. It is nevertheless preferred, that the crosslinkable scaffold precursor is stabilized by the mould for anywhere between 1-60 min during which crosslinking can occur.

In a further aspect, the present invention pertains to a crosslinked 3-dimensional (3D) scaffold for tissue engineering obtainable from a mould according to any embodiment of the present invention, or by means of the method according to the present invention, comprising encapsulated cells. An advantage of the present aspect is that the scaffold provides for good cell viability following cell encapsulation, which is advantageous for a bottom-up strategy in tissue engineering. The traditional, top-down approach involves seeding cells into full sized porous scaffolds to form tissue constructs. This approach poses many limitations such as slow vascularization, diffusion limitations, low cell density and non-uniform cell distribution. In contrast, the modular or bottom-up approach involves assembling small, non-diffusion limited, cell-laden modules to form larger structures and has the potential to eliminate the shortcomings of the traditional approach

In yet a further aspect, the present invention pertains to the use of the mould according to the present invention or of the scaffold according to the present invention in the context for tissue engineering purposes.

According to yet a further embodiment of the present invention, the use is for cell encapsulation purposes.

EXPERIMENTAL PART

In accordance with the present invention, an overview of the process going from CAD design to scaffold can be seen in *Figure 1*.
The transmittance of PVA, HIPS (High Impact Polystyrene) and Hydrosoluble were assessed compared to a blank measurement. It could be observed that no light could be transmitted through the HIPS material, furthermore, the PVA showed a transmittance of 99%. The Hydrosoluble resulted in a transmittance of 72%.
Similar results could be obtained assessing the UV intensity, namely, less than 0.1 mW/cm$^2$ loss of intensity for the PVA could be observed (8.9mW/cm$^2$ compared to 9mW/cm$^2$). The Hydrosoluble and HIPS filament resulted in a loss of 50% (4.5mM/cm$^2$) and 87% (1.2mW/cm$^2$) of the intensity respectively.

To assess the potential of the obtained scaffolds to mimic the aqueous cellular environment and assess the mass swelling ratio of the hydrogel scaffolds following removal of the mould, a mass swelling assay was performed. It could be observed that all different hydrogel materials could swell to a high extent, as can be seen in Figure 2A, maintaining previously observed differences in swelling characteristics between the material used as well as the crosslinking chemistry. Moreover, the scaffolds' mechanical properties, namely the Young's Modulus, varied between 0.8-1.9 kPa (Figure 2B).

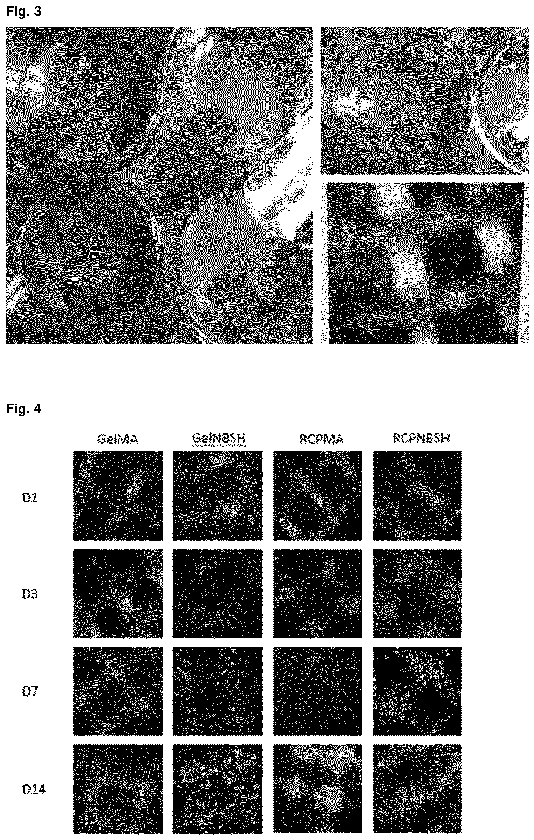

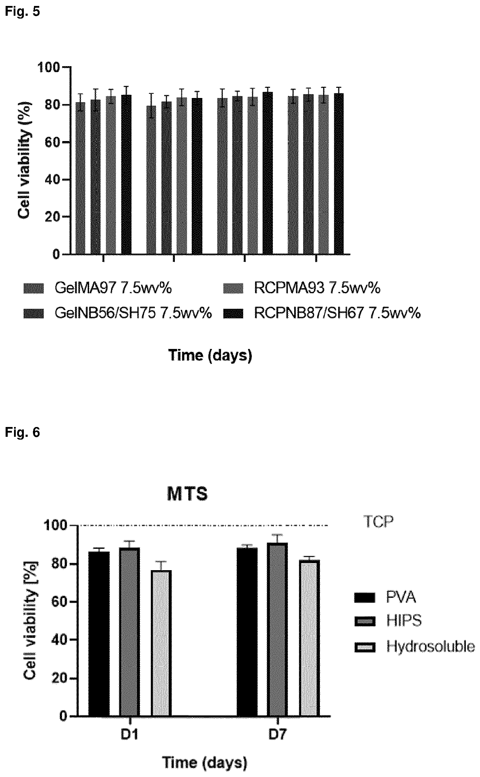

As cell encapsulation was targeted, scaffolds were made in a sterile setting, where several adipose tissue derived stem cells (ASC) containing solutions, applying the same hydrogel precursors, were injected into moulds. The obtained scaffolds can be seen in Figure 3. Next, biocompatibility of the encapsulated ASCs were assessed through live/dead staining, as can be seen in Figure 4. Here, the obtained scaffolds were assessed over a time period of 14 days, showing a high cell viability >80 % throughout all scaffolds developed (Figure 5).

Lastly, toxicity testing of the sacrificial mould compounds was performed through an indirect leaching assay. To this end, an MTS assay was performed following 24h of incubation in the 1 and 7 days leached media. All materials were biocompatible according to ISO10993 as reflected by a cell viability >70%.

**MATERIALS AND METHODS**

**MATERIALS**

The PVA filament was purchased from Ultimaker and has a PVA concentration of at least 96%. The HIPS filament was purchased from 123-3D (Almere, The Netherlands). The Hydrosoluble filament was obtained from INNOVATEFIL Smart Materials. The gelatin (type B), isolated from bovine skin through an alkaline process was kindly provided by Rousselot. The RCP was kindly provided by Fujifilm. N-(3-dimethylaminopropyl)-N'-ethylcarbodiimide (EDC), N-acetyl-homocysteine thiolactone, ethylene diamine tetra-acetic acid (EDTA) and 5-norbornene-2-carboxylic acid were purchased from Sigma-Aldrich (Diegem, Belgium). Dimethyl sulfoxide (DMSO) and N-hydroxysuccinimide (NHS) were bought from Acros (Geel, Belgium). The Spectrapor dialysis membranes MWCO 12.000-14.000 Da were purchased from Polylab (Antwerp, Belgium).

Dulbecco's modified eagle's medium (DMEM), Glutamax, Fetal bovine serum (FBS), 1% Penicillin/Streptomycin, and TrypLE (consisting of 0.025 Trypsin and 0.01 EDTA) were obtained from Gibco, Life technologies (California, USA). The ASCs (SCC038), (Calcein-acetoxymethyl ester (Calcein-AM), propidium iodide (PI), and MTS kit staining were supplied by Sigma-Aldrich.

**METHODS**

Development of Gel-MA and RCP-MA

100 g of Gel-B (38.5 mmol primary amines) was dissolved in 1 L phosphate buffer (pH 7.8) at 40 °C under continuous mechanical stirring. Next, 2.5 equivalents (eq) with respect to the primary amines present in gelatin (96.25 mmol, 14.34 ml) of methacrylic anhydride were added and the mixture was allowed to react for 1 h. Subsequently, 1 L of Milli-Q (double distilled water) was added to the reaction mixture, followed by dialysis (Spectrapor MWCO 12 000 - 14 000 Da) in Milli-Q during 24 h at 40 °C. During dialysis, the water bath was changed 5 times. Then, the pH of the solution was adjusted to 7.4 using a 1M sodium hydroxide (NaOH) solution. Finally, the Gel-MA solution was frozen (- 20 °C) and lyophilized (- 90 °C; 0.37 mbar).

The same method was used to develop RCP-MA (66.4 mmol primary amines). Briefly, 2.5 eq (166 mmol, 24.7 ml) of methacrylic anhydride was added to the mixture. The next development steps were similar as for the development of Gel-MA.

Development of Gel-NB and RCP-NB

2.5 eq of norbornene-2-carboxylic acid (14.438 mmol, 1.995 g) were dissolved in 75 ml dry dimethyl sulfoxide (DMSO) at room temperature. After complete dissolution, 2 eq of 1-Ethyl-3-(3-dimethylaminopropyl)carbodiimide hydrochloride (EDC.HCl) (11.55 mmol, 2.214 g) and 3 eq of N-Hydroxysuccinimide (NHS) (17.325 mmol, 1.994 g) were added to the reaction mixture. The solution was placed under inert argon atmosphere and allowed to react for 25 h. After 24 h, the activated mixture was heated to 50 °C for 1 h. At the same time, 15 g Gel-B was dissolved in 225 ml DMSO. After 1 h, the mixture was added to the gelatin solution to react for at least 12 h at 50 °C, under inert argon atmosphere and under continuous mechanical stirring. Next, the reaction mixture was precipitated in a tenfold excess of acetone. The precipitate was filtered using a Büchner filter (pore size: 12-15 μm) and washed again with acetone. Afterwards, the precipitate was dissolved in Milli-Q, and subsequently dialysed for 24 h at 40°C. During dialysis, the water bath was changed 5 times. After dialysis, the pH of the solution was adjusted to 7.4 using a 1M NaOH solution. Finally, the Gel-NB solution was frozen (- 20 °C) and lyophilized (-90 °C; 0.37 mbar).

The same method was used to develop RCP-NB. Briefly, 2.5 eq of norbornene-2-carboxylic acid (24,9 mmol, 3,490 g) were dissolved in 75 ml dry DMSO at room temperature. After complete dissolution, 2 eq of EDC.HCL (19,92 mmol, 3,579 g) and 3 eq of NHS (29,88 mmol, 3,439 g) were added to the mixture. The next development steps were similar as for the development of Gel-NB.

The modification of gelatin with norbornene functionalities was quantified via $^1$H-NMRspectroscopy. A Bruker WH 500 MHz spectrometer was used with deuterium oxide ($D_2O$) as solvent at 40 °C. The characteristic peaks of norbornene (at 6.0 and 6.3 ppm) were compared with a reference peak, namely the chemically inert hydrogen atoms of Val, Leu, and Ile at 1.01 ppm.

Thiolation of gelatin (Gel-SH) and RCP (RCP-SH)

20 g Gel-B was added to 200 ml carbonate buffer (pH 10) at 40 °C and allowed to dissolve under inert argon atmosphere. Next, 90.4 mg (1,5 mM) ethylene diamine tetra-acetic acid (EDTA) was added to prevent the thiol groups from reacting with each other. Afterwards, 5 eq of N-acetyl homocysteine thiolactone (38.5 mmol, 6.193 mg) were added and allowed to react for 3 h at 40 °C, under inert argon atmosphere and under continuous mechanical stirring. Gel-SH was purified using dialysis for 24 h under inert argon atmosphere. During dialysis, the water bath was changed 5 times. Finally, the Gel-SH solution was frozen (- 20 °C) and lyophilized (-90 °C; 0.37 mbar).

The same method was used to develop RCP-SH. Briefly, 5 eq of N-acetyl homocysteine thiolactone (66.4 mmol, 10.5 mg) were added to the mixture. The next development steps were similar as for the development of Gel-SH.

A spectrophotometric o- phthaladehyde (OPA) assay was used to determine the degree of substitution (DS) of the developed thiolated gelatin.26 The assay is based on the reaction between amine groups with o-phthaladehyde (OPA) and 2-mercaptoethanol. The reaction is specific toward the primary amines and the absorption of the OPA adducts is measured at 335 nm at 37 °C. The samples were evaluated in triplicate.

Scaffold preparation

To determine the physio-chemical properties, Gel-MA (DS97), Gel-NB-SH (DS56 and 75 respectively), RCP-MA (DS93), and RCP-NB-SH (DS87 and 75 respectively) scaffolds were made. To this end, a 7.5 w/v% aqueous solution was prepared of the different hydrogel precursors and 0.6mM lithium phenyl-2,4,6-trimethylbenzoylphosphinate (LAP) was added.

The PVA moulds were produced via fused deposition modelling (Ultimaker 3). The scaffolds exhibited a grid-like structure with the following dimensions: $10 \times 10 \times 10$ mm$^3$ and an intended pore size of 1000 $\mu$m. The precursor solution was then injected into the pouring cup before crosslinking occurred physically (15 min at 6°C in the dark) followed by chemical crosslinking (UV-A, 365nm, 9 mW/cm$^2$, 30 min). The moulds were washed away by submerging them in PBS at 37°C for several hours.

Translucency

A UV-Vis spectrophotometer (Uvikon XL, BioTek Instruments) was used to test the transmittance of a film constituting the filament material (0.5 mm thickness) combined with PBS. The wavelength of the light beam ranged from 300 nm to 800 nm. The loss in transmittance was calculated compared to a blank measurement.
Furthermore, the UV intensity of 1 mm thick films made of the proposed filament materials were assessed with a dosimeter, and compared to the UV-A intensity without any films between the lamps and the dosimeter.

Mass swelling ratio

Scaffolds were weighed after incubation at 37 °C in double-distilled water. Before weighing the equilibrium-swollen samples, the surface was gently dipped with a paper towel to remove surface water. The equilibrium mass swelling ratio was defined as:

$$\text{mass swelling ratio (Q)} = Ws/Wd$$

where Ws is the mass after 24 h of swelling at 37 °C and Wd is the freeze-dried mass following incubation and swelling.

Mechanical properties

Tinius Olsen 5ST (Horsham, USA) was used to analyze the mechanical strength of the obtained scaffolds. First, scaffolds were equilibrium swollen in double distilled water (ddH$_2$O). A caliper was used to measure the dimensions of the swollen scaffolds. Next, the stress of the scaffolds was assessed using a load cell of 25N. The stress and strain were recorded at a constant speed of 10 mm/min. The Young's modulus was calculated via the slope of the first linear part of obtained plot depicting stress as a function of strain.

Cell Encapsulation Experiments

For all experiments, ASCs were used in passages 3-5. Next, a 7.5 w/v % solution of the hydrogel precursors was made in phosphate-buffered saline (PBS). When fully dissolved, 1.000.000 cells were added per mL of solution and mixed before the photo-initiator (0.6mM LAP) was added: to each of the moulds -400 $\mu$L of the cell-polymer solution containing PI, was injected. The UV setup was placed in a laminar flow cabinet. Following 10 min of physical gelation in the fridge, the scaffolds were exposed for 15 min to UV light at a high intensity (365 nm, 7 mW/cm$^2$). After crosslinking, 1 mL of the basic medium (DMEM, 10% FBS, and 1% penicillin/streptomycin) was added to each well to provide nutrients to the cells and dissolve the mould. The well plate was placed in an incubator at 37 °C and in the presence of 5% CO$_2$. The medium was refreshed every 2-3 days.

In vitro biocompatibility assays

- *Live/dead cell viability assay*

The cytocompatibility of cell-encapsulating materials obtained above was tested through a live/dead viability assay using Calcein acetoxymethyl ester (Calcein-AM) and propidium iodide (PI) staining. For every 1 mL PBS, 2 $\mu$l Calcein-AM and 2 $\mu$l PI were added. A 96-well plate was used and 0.15 mL of the solution was added to each well. The wells were incubated in the dark (protected from light with aluminum foil) for 10 minutes at room temperature. A fluorescence microscope with a green fluorescent protein (GFP) filter for Calcein was used to visualize the living cells. A Texas Red (TxRed) filter was applied to visualize the dead cells using PI. Quantification of the live/dead ratio was realized via Imaged software, enabling a cell count of both living and dead cells.

*- Metabolic activity assays*

An 3-(4,5-dimethylthiazol-2-yl)-5-(3-carboxymethoxy phenyl)-2-(4-sulfophenyl)-2H-tetrazolium (MTS) assay kit was used to assess the cytotoxicity of the different filaments described in the patent. The filaments were incubated in cell culture media for 1 and 7 days (250 mg/mL), prior to placing the media on cells seeded on tissue culture plates (TCP) (density of 30.000 cells/well). To this end, the basic culture medium of the cells was discarded and refreshed with 250 $\mu$L of the leached media. The cells were incubated with the solution for 24h, before removal of the media. Next, 200$\mu$L of fresh basic culture media was added to the well plates with the MTS solution (40 $\mu$L in a 48 well plate). The well plates were incubated in the dark (covered in aluminum foil) on a shaker at 37 °C for 2h. Next, 100 $\mu$L of the supernatant was added to a 96 well plate. Lastly, the 96 well plate was placed on a plate reader measuring the absorbance (at 580 nm) using a spectrophotometer (Biotek EL-800).

**Claims**

1. A mould for casting a crosslinked 3-dimensional scaffold for tissue engineering, wherein the mould is for receiving a crosslinkable scaffold precursor in a liquid state, and wherein the mould is made of a water-soluble material.

2. The mould according to the previous claim, wherein the water-soluble material has a solubility in water at 37ºC from 0.001 g/ml to 1 g/ml, preferably from 0.05 g/ml to 0.75 g/ml, more preferably 0.25 g/ml to 0.5 g/ml.

3. The mould according to any one of the previous claims, wherein the mould is of a material having a total transmittance of at least 80% at a wavelength from 300nm to 800nm upon contact with the crosslinkable scaffold precursor, preferably a total transmittance of at least 85%, preferably at least 90%, more preferably at least 95%.

4. The mould according to any one of the previous claims, wherein the water-soluble material comprises PVA.

5. The mould according to any one of claims 5 , wherein the mould has a minimum thickness $T_{min}$, measured as the shortest distance from a point A on an inner surface to a point B on an outer surface, wherein $T_{min}$ is from 100 to 1500$\mu$m, preferably from 150 to 1250$\mu$m, more preferably from 250 to 1000$\mu$m.

6. The mould according to any one of the previous claims, further comprising a pouring cup adapted to receive a crosslinkable scaffold precursor.

7. A method of manufacturing of a crosslinked scaffold for tissue engineering comprising the steps of:

   a) providing a mould of a water-soluble material as described in any one of claims 1 to 6;
   b) providing a crosslinkable scaffold precursor in a liquid state;
   c) casting the crosslinkable scaffold precursor into the mould provided at step a), thereby obtaining a casted crosslinkable scaffold precursor;
   d) crosslinking the casted crosslinkable scaffold precursor obtained at step c), thereby obtaining the crosslinked scaffold precursor,
   e) providing a solvent adapted to dissolve the mould provided at step a);
   f) removing the mould from the crosslinked scaffold at step d) by means of dissolution of the mould provided at step a) with the solvent provided at step e), thereby obtaining the crosslinked scaffold.

8. The method of manufacturing according to claim 7, wherein at step b) the crosslinkable scaffold precursor comprises one or more of: cells, stem cells, organoids, spheroids, cell clusters, co-cultures.

9. The method of manufacturing according to any one of claims 7 to 8, wherein at step b) the crosslinkable scaffold precursor has a water content from 2.5 to 15 w/v%, preferably from 5 to 12.5, more preferably from 5 to 10.

10. The method of manufacturing according to any one of claims 7 to 9, wherein at step a) the mould is made of a material which is transparent or translucent after the crosslinkable scaffold precursor has been received by the mould, allowing radiation, preferably UV-vis radiation, to reach the crosslinkable scaffold precursor.

11. The method of manufacturing according to claim 10, wherein at step a) the mould is made of a material comprising PVA.

12. The method of manufacturing according to any one of claims 7 to 11, wherein at step b) the crosslinkable scaffold precursor has a viscosity from 0.05 to 10 mPa.s, preferably from 0.5. to 1.5 mPa.s.

13. The method of manufacturing according to any one of claims 7 to 12, wherein at step d) the crosslinking is performed from about 1 to 30 min, preferably from about 1 to 20 min.

14. A crosslinked 3-dimensional scaffold for tissue engineering obtainable from a mould according to any one of claims 1 to 6 or by means of the method according to claims 7 to 13, comprising encapsulated cells.

15. Use of the mould according to any one of claims 1 to 6 or of the scaffold according to claim 14 for tissue engineering purposes, such as for cell encapsulation purposes.

**Fig. 1**

A          B          C

**Fig. 2**

A

B

**Fig. 3**

**Fig. 4**

**Fig. 5**

**Fig. 6**

# EUROPEAN SEARCH REPORT

Europäisches Patentamt
European Patent Office
Office européen des brevets

**Application Number**

**EP 22 21 4186**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/028565 A1 (SINOBIOPRINT SHANGHAI BIOTECH LTD [CN]) 10 February 2022 (2022-02-10) * claims 1, 2, 7: example 4 * ----- | 1-15 | INV. A61L27/16 A61L27/22 A61L27/24 A61L27/38 |
| A | PIAO YUN ET AL: "Biomedical applications of gelatin methacryloyl hydrogels", ENGINEERED REGENERATION, vol. 2, 1 January 2021 (2021-01-01), pages 47-56, XP93047805, ISSN: 2666-1381, DOI: 10.1016/j.engreg.2021.03.002 * abstract * ----- | 1-15 | A61L27/50 A61L27/52 B33Y70/00 B33Y80/00 |

**TECHNICAL FIELDS SEARCHED (IPC)**

A61L
B33Y

The present search report has been drawn up for all claims

| Place of search | Date of completion of the search | Examiner |
|---|---|---|
| The Hague | 17 May 2023 | Cadamuro, Sergio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

16

## ANNEX TO THE EUROPEAN SEARCH REPORT
## ON EUROPEAN PATENT APPLICATION NO.

**EP 22 21 4186**

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2023

| Patent document cited in search report | Publication date | Patent family member(s) | Publication date |
|---|---|---|---|
| WO 2022028565 A1 | 10-02-2022 | NONE | |

EPO FORM P0459